**Europäisches Patentamt**

**European Patent Office**

**Office européen des brevets**

(19)

(11) Veröffentlichungsnummer: **0 144 367**

**B1**

(12) # EUROPÄISCHE PATENTSCHRIFT

(45) Veröffentlichungstag der Patentschrift:
**29.03.89**

(21) Anmeldenummer: **84901995.5**

(22) Anmeldetag: **17.05.84**

(86) Internationale Anmeldenummer:
**PCT/EP 84/00148**

(87) Internationale Veröffentlichungsnummer:
**WO 84/04817 (06.12.84 Gazette 84/28)**

(51) Int. Cl.⁴: **G 01 N 33/50**, G 01 N 33/92, G 01 N 33/53

(54) **VERFAHREN UND REAGENZ ZUM DIREKTEN NACHWEIS EINES ANALYTS NACH SPEZIFISCHER ENTFERNUNG VON COLLOIDPARTIKELN IN WÄSSRIGEN LÖSUNGEN BIOLOGISCHER HERKUNFT.**

(30) Priorität: **26.05.83 DE 3319066**

(43) Veröffentlichungstag der Anmeldung:
**19.06.85 Patentblatt 85/25**

(45) Bekanntmachung des Hinweises auf die Patenterteilung:
**29.03.89 Patentblatt 89/13**

(84) Benannte Vertragsstaaten:
**AT BE CH DE FR GB IT LI LU NL SE**

(56) Entgegenhaltungen:
**EP-A-0 008 338**
**EP-A-0 092 801**
**WO-A-80/02460**
**FR-A-2 381 311**

**Chemical Abstracts, volume 94, No. 15, 13 April 1981, Columbus, Ohio (US). W. Stoffel et al.: "Selective removal of apolipoprotein B - containing serum lipoproteins from blood plasma", see page 479, column 2, abstract No. 118919a & Proc. Nat. I. Acad. Sci. U.S.A. 1981, 611-615 (Eng)**

(73) Patentinhaber: **BOEHRINGER MANNHEIM GMBH, Sandhofer Strasse 116, D-6800 Mannheim 31 (DE)**

(72) Erfinder: **HEUCK, Claus- Christian, Birkenweg 20, D-6901 Wilhelmsfeld (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren und ein Reagenz zur direkten Bestimmung von in LDL- bzw. in HDL enthaltenen Lipiden in Körperflüssigkeiten.

Die wasserunlöslichen Fette, Triglyceride, Cholesterin und Phospholipide, werden im wässrigen Milieu des menschlichen Plasma in mizellähnlichen Bestandteilen, den Serumlipoproteinen, transportiert. Es können verschiedene Lipoproteinklassen im menschlichen Nüchternserum nachgwiesen werden. Sie alle enthalten Triglycerid, Cholesterin, Phospholipid und bestimmte Proteine, sogenannte Apolipoproteine. Die drei im menschlichen Serum nachweisbaren, großen Lipoproteinklassen unterscheiden sich in ihrer spezifischen Dichte. Sie werden infolge dessen als Very Low Density Lipoproteine (VLDL), Low Density Lipoproteine (LDL) und High Density Lipoproteine (HDL) bezeichnet. Die Apolipoproteine werden ebenfalls in verschiedene Klassen unterteilt, die mit einem Buchstaben gekennzeichnet sind (z. B. Apolipoprotein A (Apo-A), Apolipoprotein B (Apo-B), Apolipoprotein C (Apo-C), Apolipoprotein D (Apo-D) und Apolipoprotein (Apo-E). Kennzeichnenderweise sind die Apolipoproteinklassen meist nicht auf eine sondern mehrere Lipoproteinklassen verteilt. So enthalten die VLDL Apo-A, Apo-B, Apo-C und Apo-E, die HDL enthalten Apo-A, Apo-C und Apo-E. Hingegen enthalten die LDL nahezu ausschließlich Apo-B.

Die pathognomonische Bedeutung der einzelnen Lipoproteinklassen ist für die Ablagerung von Fetten, insbesondere von Cholesterin, in den Gefäßwänden unterschiedlich. Die LDL bestehen zum größten Teil aus Cholesterin. Ihr Beitrag zur Ablagerung von Cholesterin in den Gefäßen - ein Prozeß, der als Atherogenese bezeichnet wird - ist nachgewiesenermaßen am größten. Die HDL sind Phospholipid-reich. Sie vermögen Cholesterin aus den Zellen der Gefäße aufzunehmen und abzutransprotieren. Ihnen wird daher eine antiatherogene Wirkung nachgesagt.

In klinisch-chemischen Untersuchungen wurde herausgefunden, daß Personen mit erhöhten LDL-Konzentrationen im Plasma oder Serum ein höheres Risiko haben, an Herz-Kreislauferkrankungen zu leiden, während Personen mit erhöhten HDL-Konzentrationen deutlich weniger anfällig sind. Es hat sich daher für die labormedizinische Diagnostik als vorteilhaft erwiesen, nicht nur die Bestimmung von Gesamtcholesterin im Serum sondern auch die Bestimmung von Cholesterin in der LDL-Fraktion bzw. in der HDL-Fraktion zur prognostischen Beurteilung des Ausmaßes der Gefährdung einer Person in Betracht zu ziehen. Insbesondere der Bestimmung von Cholesterin in den LDL wird eine herausragende Bedeutung beigemessen.

Die Bestimmung von LDL-Cholesterin aus Plasma oder Serum wird herkömmlicherweise mit Hilfe kombinierter Verfahren, z. B. der Ultrazentrifugation und Polyanionenpräzipitation in Speziallaboratorien (Lipid Research Clinics Program, DHEW Publication No (NIH) 75 - 628, Bethesda, Md. (1974)) oder aus den Konzentrationen von Gesamtcholesterin, Gesamttriglycerid und HDL-cholesterin im Serum durch Berechnung mit Hilfe der Formel nach Friedewald (Clin. Chem. 18, 499 (1972) durchgeführt. Außerdem werden bei der Bestimmung von LDL-Cholesterin die Präzipitation aufgrund spezifischer Immunreaktionen verwendet (FR-A-2 381 311; EP-A-0 008 338; EP-A-0 092 801). Der Nachteil dieser Methoden ergibt sich aus der indirekten Bestimmung der Meßgröße, da hierbei die Fehler aller Messungen in die Berechnung des Wertes für LDL-Cholesterin einfließen.

Beispielsweise erfolgt sowohl nach FR-A-2 381 311 als auch nach EP-A-0 008 338 die Bestimmung des Lipids nicht direkt, sondern über entsprechende Serumproteine durch optische Analysenmethoden nachdem interferierende Lipoproteinfraktionen abgetrennt wurden.

Die französische Patentanmeldung 2 381 311 offenbart ein Verfahren für die turbidimetrische Bestimmung des nur bei bestimmten Krankheiten vorliegenden Lipoprotein X in Serumproben, aus denen zuvor VLDL- und LDL-Partikel durch den Zusatz von gegen Apo B gerichtete Antikörper entfernt wurden.

Nach EP-A-0 008 338 werden vor der turbidimetrischen Bestimmung spezifischer Serumproteine störende Lipoproteinfraktionen durch ein entsprechendes immunaktives Reagenz und den Zusatz von hydrolytischen Enzymen entfernt. Der Zusatz der Enzyme erfolgt hier zum Zweck einer normierten Lipoprotein-Bestimmung und bewirkt eine Vereinheitlichung in der Größe bzw. in den Volumina der zu bestimmenden Lipoproteinpartikel.

Die ältere europäische Anmeldung 0 092 801 offenbart dagegen ein Verfahren zur Bestimmung von in LDL enthaltenen Lipiden, wobei die interferierenden Lipoproteinfraktionen hier jedoch durch den Zusatz von HDL-Antikörper, nicht durch Antikörper gegen isolierte Apolipoproteine, abgetrennt werden. Das Verfahren gemäß EP-A-92 801 gehört zum Stand der Technik gemäß Artikel 54 (3) EPÜ.

Gegenstand der Erfindung ist ein Verfahren zur direkten Bestimmung von in LDL enthaltenen Lipiden in Körperflüssigkeiten, wobei Antikörper gegen Apolipoprotein A oder C oder Antikörper gegen die Apolipoproteine A und C und ein oder mehrere Enzyme hinzugefügt werden, die bewirken, daß die kolloidchemische Stabilität der zu entfernenden Fraktionen vermindert wird, und anschließend der direkte Nachweis des Lipids geführt wird;

ein Verfahren zur direkten Bestimmung von in HDL enthaltenen Lipiden in Körperflüssigkeiten, wobei zur untersuchenden Probe Antikörper gegen Apolipoprotein B und ein oder mehrere Enzyme hinzugefügt werden, die bewirken, daß die kolloidchemische Stabilität der zu entfernenden Fraktionen vermindert wird und anschließend der direkte Nachweis des Lipids geführt wird;

ein Reagenz zur direkten Bestimmung von in LDL enthaltenen Lipiden in Körperflüssigkeiten, wobei es einen oder mehrere Antikörper gegen Apolipoprotein A oder C oder gegen die Apolipoproteine A und C und ein oder mehrere Enzyme enthält, die bewirken, daß die kolloidchemische Stabilität der zu entfernenden Fraktionen vermindert wird, und

ein Reagenz zur direkten Bestimmung von in HDL enthaltenen Lipiden in Körperflüssigkeiten, wobei es einen oder mehrere Antikörper gegen Apolipoprotein B und ein oder mehrere Enzyme enthält, die bewirken, daß die kolloidchemische Stabilität der zu entfernenden Fraktionen vermindert wird.

Überraschenderweise wurde festgestellt, daß die direkte Bestimmung von Lipiden, z. B. Cholesterin, in den bestimmten oben genannten Lipoproteinfraktionen, in hervorragender Weise ausgeführt werden kann, wenn aus der zu untersuchenden Probe die Lipoproteinpartikel, die zwar auch Lipide enthalten, jedoch nicht der zu untersuchenden Lipoproteinfraktion zugeordnet sind, durch die spezifische Immunreaktion entfernt werden. Das Verfahren setzt voraus, daß das Reagenz immunchemische Eigenschaften hat, die ausschließlich gegen die zu entfernenden Lipoproteine gerichtet sind, und ein oder mehrere die kolloidchemische Stabilität der zu entfernenden Lipoproteinpartikel herabsetzende Enzyme enthält. Das Reagenz mit immunchemischen Eigenschaften gegen Apo A und Apo C oder mit immunchemischen Eigenschaften gegen Apo A oder Apo C reagiert mit VLDL- und HDL-Partikeln, die beide Apo A und Apo C enthalten. Es reagiert hingegen nicht mit LDL-Partikeln, die diese Apolipoproteine nicht enthalten. Entsprechendes gilt für HDL-Partikel und Apo B enthaltene Lipoproteinfraktionen. Mit Hilfe einer von den oben genannten spezifischen immunchemischen Reaktionen können die in einer Probe vorhandenen verschiedenen Lipoproteinklassen voneinander getrennt werden. Der Gehalt an Lipiden in der Restlösung entspricht somit dem Lipidgehalt der nicht umgesetzten Lipoproteinfraktionen unter der Voraussetzung, daß das Immunreagenz Lipide gleicher Art in Mengen enthält, die die Messung nicht beeinträchtigen.

Aus der Restlösung kann daher der Lipidgehalt - gegebenenfalls unter Berücksichtigung eines Leerwertes - direkt gemessen werden. Da das Plasma aller Tierspezies, die zur Antikörpersynthese immunisiert werden, physiologischerweise Lipide enthält, die den quantitativen Nachweis eines entsprechenden Analyts in einem Humanserum beeinträchtigen würden, müssen diese zuvor aus dem Immunreagenz entfernt werden. Wie dem Fachmann vertraut ist, findet die Immunpräzipitation zwischen 4° C und 42° C in einem pH-Milieu zwischen 5 und 9, vorzugsweise bei 25° C und pH 7 bis 8,6 statt.

Wie anhand von Vergleichsuntersuchungen festgestellt wurde, stimmt die in der dargelegten Weise durchgeführte Analytik der direkten Bestimmung z. B. von Cholesterin in den LDL oder HDL aus Serum oder Plasma menschlicher Herkunft mit normalen bzw. nur leicht erhöhten Lipidkonzentrationen auch ohne Zusatz eines oder mehrerer Enzyme der genannten Art hervorragend mit den Messungen mit Hilfe der herkömmlichen Verfahren überein. Bei höheren Lipidkonzentrationen, insbesondere bei höheren Triglyceridkonzentrationen ist diese Trennung der Lipoproteinfraktionen in vereinzelten Seren oder Plasmen unvollständig, wenn die Fraktionierung durch eine Immunpräzipitation mit einem Reagenz, das nicht an eine Festphase gekoppelt ist, ausgeführt wird. Es hat sich daher in solchen Fällen als besonders vorteilhaft erwiesen, wenn die Präzipitation in Gegenwart von einem oder mehreren Enzymen, die die kolloidchemische Stabilität der zu entfernenden Lipoproteinpartikel herabsetzen, ausgeführt wird. Durch die gleichzeitige enzymatische Behandlung können noch gelöste Immunkomplexe ebenfalls präzipitiert werden.

Die Verwendung verschiedener Enzyme hat sich hierfür als vorteilhaft erwiesen:

| EC 3.2.1.18 | Neuraminidase |
| EC 3.4.22.2 | Papain |
| EC 3.4.21.4 | Pronase |
| EC 3.1.4.12 | Sphingomyelinase |
| EC 3.1.4.3 | Phospholipase C |
| EC 3.1.1.13 | Cholesterinesterase |
| EC 3.1.1.3 | Triglyceridlipase |
| EC 3.1.1.1. | Carboxylesterase |

Die notwendigen Mengen der Enzyme für eine Bestimmung sind durch die Art der Immunpräzipitation und durch Spezifität und Aktivität des jeweiligen Enzyms bestimmt.

Das Reagenz ist zur selektiven Entfernung von störenden Bestandteilen in einer wässrigen Lösung durch eine Immunreaktion zum Zwecke des nachfolgenden direkten Nachweises des genannten Analyts in der Flüssigkeit bestimmt. Den nachzuweisenden Analyt enthält das Reagenz nicht oder nur in Mengen, deren Beitrag für den quantitativen Nachweis der Konzentrationen des Analyts in einer Probenlösung rechnerisch berücksichtigt werden kann. Die Spezifität der Antikörper richtet sich, wie sich schon aus der Darlegung zum Verfahren ergibt, gegen die jeweiligen störenden und damit zu entfernenden Bestandteile in der Probenlösung. Die Antikörper können hierbei in dem Reagenz in Lösung vorliegen oder auch an eine Festphase gebunden sein.

Für die Entfernung der störenden Bestandteile einer Probenlösung durch Immunpräzipitation enthält das Reagenz ein oder mehrere Enzyme, die die Oberflächeneigenschaften der störenden Bestandteile in dem Sinne verändern, daß eine Immunpräzipitation begünstigt wird. Die verwendeten Enzyme entsprechen denen, die in der Darlegung der Ausführung des Verfahrens vermerkt sind. Im Falle der Aufbereitung der Probe durch Immunadsorption haben sich die dem Fachmann vertrauten Festphasen zur Fixation der Antikörper (Zellulose, Sepharose, Dextran, Polyakrylamid, controlled pore glass etc) als anwendbar erwiesen.

Die folgenden Beispiele sollen die Vorteile des Verfahrens verdeutlichen:

### Beispiel 1: (Vergleichsversuch)

Jeweils 5 µl von triglyceridarmen Humanseren wurden mit jeweils 50 µl eines delipidierten Serums vom Kaninchen mit Antikörpern gegen Human-Apolipoprotein A durchmischt. Nach einer Stunde Aufbewahrung bei Raumtemperatur wurde die Mischung in einer Laborzentrifuge zentrifugiert und der Cholesteringehalt in der überständigen Lösung mit einer gebräuchlichen enzymatischen Meßmethode ermittelt. Aus den gleichen Seren wurde der Gehalt an LDL-Cholesterin mit Hilfe der herkömmlichen Technik der Ultrazentrifugation und Polyanionenpräzipitation sowie mit Hilfe der Methode nach Friedewald gemessen. Die Ergebnisse der Messungen von verschiedenen Serumproben sind in der Tabelle 1 zum Vergleich aufgeführt.

### Beispiel 2:

66 verschiedene Seren mit einem normalen Gehalt oder pathologisch erhöhten Gehalt an Cholesterin und/oder Triglycerid wurden mit einem delipidierten Antiserum, das Antikörper gegen menschliche Apolipoproteine A und C und Neuraminidase (10 mU) enthielt, wie in Beispiel 1 beschrieben untersucht. Die Ergebnisse wurden mit der LDL-Cholesterinbestimmung nach der Methode von Friedewald verglichen. Der Korrelatonsfaktor zwischen beiden Bestimmungsmethoden betrug:

r: 0,97 bei einer Regression von
y = 0,89 x + 0,078

(y: Cholesterinkonzentration (g/l) nach Immunpräzipitation; x: Cholesterinkonzentration (g/l) nach Berechnung aus der Friedewaldformel).

### Beispiel 3:

In gleicher Weise wie in Beispiel 1 beschrieben wurden Humanseren mit einem delipidierten Antiserum gegen Human-Apo B in Gegenwart von Triglyceridlipase (500 mU) und Carboxylesterase (5 U) vermischt. Nach einer Stunde wurde die Mischung zentrifugiert und im Überstand der Gehalt an Cholesterin gemessen. Die Ergebnisse sind in Tabelle 2 mit Referenzverfahren der HDL-Cholesterinbestimmung nach Polyanionenpräzipitation aus dem Vollserum verglichen.

Die Ergebnisse der angeführten Beispiele veranschaulichen, daß mit Hilfe der verschiedenen Variationen des entwickelten Verfahrens präzise direkte Messungen von Cholesterin in einer Lipoproteinklasse des menschlichen Serums, die nicht mit einem lipidfreien Reagenz mit bestimmten immunologischen Eigenschaften reagiert, aus minimalen Probenvolumina ohne aufwendige labortechnische Hilfsmittel möglich sind. Selbstverständlich gelingt auch die Bestimmung des Gehaltes von anderen Lipiden, z. B. Triglycerid oder Phospholipid, in der gleichen Lipoproteinfraktion, da auch diese Lipide in dem Reagenz nicht in störender Weise enthalten sind. Das Verfahren bietet somit beträchtliche Vorteile, z. B. für die klinisch-chemische Differentialdiagnostik von Fettstoffwechselstörungen; insbesondere ist die neubeschriebene Analytik wesentlich einfacher und kostengünstiger als die herkömmlicherweise eingesetzten Verfahren.

Das Prinzip des am Beispiel der Analytik von Serumlipoproteinen erläuterten Verfahrens kann in analoger Weise durch Verwendung eines Reagenz mit geeigneten spezifischen immunologischen Merkmalen zur Untersuchung eines Analyts in anderen Flüssigkeiten biologischer Herkunft (z. B. Zellkulturmedium, Urin, Liquor etc.), der an Biopolymere mit unterschiedlichen immunogenen Eigenschaften gebunden ist, problemlos zu analytischen oder präparativen Zwecken angewandt werden.

**Tabelle 1** Bestimmung von "LDL"-Cholesterin

| I | II | III |
|---|---|---|
| 5,09 g/l | 5,45 g/l | 5,21 g/l |
| 1,36 " | 1,20 " | 1,19 " |
| 1,37 " | 1,53 " | 1,32 " |
| 1,48 " | 1,47 " | 1,45 " |
| 1,24 " | 1,30 " | 1,12 " |
| 1,11 " | 1,23 " | 1,19 " |
| 1,18 " | 1,10 " | 1,12 " |
| 1,45 " | 1,40 " | 1,32 " |
| 2,14 " | 2,22 " | 1,98 " |
| 1,01 " | 1,02 " | 0,95 " |

I:
LDL-Cholesterin (nach Ultrazentrifugation)
II: LDL-Cholesterin (nach Friedewald)
III: Cholesterin (nach Verfahren Beispiel 1)

**Tabelle 2** Bestimmung von HDL-Cholesterin

| Serum | I | II |
|---|---|---|
| 1 | 0,400 g/l | 0,430 g/l |
|  | 0,220 " | 0,230 " |
|  | 0,420 " | 0,460 " |
|  | 0,350 " | 0,330 " |
|  | 0,760 " | 0,820 " |
|  | 0,320 " | 0,330 " |
|  | 0,370 " | 0,630 " |
|  | 0,280 " | 0,300 " |
|  | 0,640 " | 0,730 " |
|  | 0,580 " | 0,650 " |

I Cholesterinkonzentrationen im Serum nach Fällung mit Phosphorwolframsäure/MgCl$_2$
II Cholesterinkonzentrationen im Serum nach Immunpräzipitation und Gammaglobulin gegen Apolipoprotein B in Gegenwart von Triglyceridlipase (EC 3.1.1.3) und Carboxylesterase (EC 3.1.1.1)

**Patentansprüche**

1. Verfahren zur direkten Bestimmung von in LDL enthaltenen Lipiden in Körperflüssigkeiten, dadurch gekennzeichnet, daß Antikörper gegen Apolipoprotein A oder C oder Antikörper gegen die Apolipoproteine A und C und ein oder mehrere Enzyme hinzugefügt werden, die bewirken, daß die kolloidchemische Stabilität der zu entfernenden Fraktionen vermindert wird, und anschließend der direkte Nachweis des Lipids geführt wird.

2. Verfahren zur direkten Bestimmung von in HDL enthaltenen Lipiden in Körperflüssigkeiten, dadurch gekennzeichnet, daß zur untersuchenden Probe Antikörper gegen Apolipoprotein B und ein oder mehrere Enzyme hinzugefügt werden, die bewirken, daß die kolloidchemische Stabilität der zu entfernenden Fraktionen vermindert wird und anschließend der direkte Nachweis des Lipids geführt wird.

3. Verfahren nach Anspruch 1 oder 2, dadurch gekennzeichnet, daß als Enzym Neuraminidase, Pronase, Papain, Triglyceridlipase, Carboxylesterase, Cholesterinesterase, Sphingomyelinase, Phospholipase oder ein Gemisch derselben verwendet werden.

4. Reagenz zur direkten Bestimmung von in LDL enthaltenen Lipiden in Körperflüssigkeiten, dadurch gekennzeichnet, daß es einen oder mehrere Antikörper gegen Apolipoprotein A oder C oder gegen die Apolipoproteine A und C und ein oder mehrere Enzyme enthält, die bewirken, daß die kolloidchemische Stabilität der zu entfernenden Fraktionen vermindert wird.

5. Reagenz zur direkten Bestimmung von in HDL enthaltenen Lipiden in Körperflüssigkeiten, dadurch gekennzeichnet, daß es einen oder mehrere Antikörper gegen Apolipoprotein B und ein oder mehrere Enzyme enthält, die bewirken, daß die kolloidchemische Stabilität der zu entfernenden Fraktionen vermindert wird.

6. Reagenz nach Anspruch 4 oder 5, dadurch gekennzeichnet, daß es als Enzym Neuraminidase, Pronase, Papain, Triglyceridlipase, Carboxylesterase, Cholesterinesterase, Sphingomyelinase, Phospholipase oder ein Gemisch derselben enthält.

**Claims**

1. Process for the direct determination of lipids contained in LDL in body fluids, characterised in that antibodies against apolipoprotein A or C or antibodies against the apolipoproteines A and C and one or more enzymes are added thereto which bring about that the colloid-chemical stability of the fractions to be removed is reduced and subsequently the direct detection of the lipid is carried out.

2. Process for the direct determination of lipids contained in HDL in body fluids, characterised in that antibodies against apolipoprotein B and one or more enzymes are added to the sample to be investigated which bring about that the colloid-chemical stability of the fractions to be removed is reduced and subsequently the direct detection of the lipid is carried out.

3. Process according to claim 1 or 2, characterised in that as enzyme there are used neuraminidase, pronase, papain, triglyceride lipase, carboxyl esterase, cholesterol esterase, sphingomyelinase, phospholipase or a mixture thereof.

4. Reagent for the direct determination of lipids contained in LDL in body fluids, characterised in that it contains one or more antibodies against apolipoproteins A or C or against the apolipoproteins A and C and one or more enzymes which bring about that the colloid-chemical stability of the fractions to be removed is reduced.

5. Reagent for the direct determination of lipids contained in HDL in body fluids, characterised in that it contains one or more antibodies against apolipoprotein B and one or more enzymes which bring about that the colloid-chemical stability of the fractions to be removed is reduced.

6. Reagent according to claim 4 or 5, characterised in that as enzyme it contains neuraminidase, pronase, papain, triglyceride lipase, carboxyl esterase, cholesterol esterase, sphingomyelinase, phospholipase or a mixture thereof.

**Revendications**

1. Procédé pour la détermination directe de lipides contenus dans le LDL dans les liquides biologiques, caractérisé en ce que des anticorps dirigés contre l'apolipoprotéine A ou C ou des anticorps dirigés contre les apolipoprotéines A et C, et un ou plusieurs enzymes sont ajoutés, qui agissent de façon à réduire la stabilité chimique colloïdale des fractions à éliminer, et ensuite, la détermination directe des lipides est effectuée.

2. Procédé pour la détermination directe de lipides contenus dans le HDL dans les liquides biologiques, caractérisé en ce qu'à l'échantillon examiné, des anticorps dirigés contre l'apolipoprotéine B et un ou plusieurs enzymes sont ajoutés, qui agissent de façon à réduire la stabilité chimique colloïdale des fractions à éliminer, et ensuite, la détermination directe des lipides est effectuée.

3. Procédé selon la revendication 1 ou 2, caractérisé en ce que comme enzyme, on utilise le neuramidinase, le pronase, la papaine, le triglycéridlipase, le carboxylesterase, le cholestérinesterase, le sphingomyélinase, le phospholipase ou un mélange de ces enzymes.

4. Réactif pour la détermination directe de lipides contenus dans le LDL dans les liquides biologiques, caractérisé en ce qu'il contient un ou plusieurs anticorps dirigés contre l'apoliprotéine A ou C ou des anticorps dirigés contre les apoliprotéines A et C et un ou plusieurs enzymes, qui agissent de façon à réduire la stabilité chimique colloïdale des fractions à éliminer.

5. Réactif pour la détermination directe de lipides contenus dans le HDL dans les liquides biologiques, caractérisé en ce qu'il contient un ou plusieurs anticorps dirigés contre l'apolipoprotéine B et un ou plusieurs enzymes, qui agissent de façon à réduire la stabilité chimique colloïdale des fractions à éliminer.

6. Réactif selon les revendications 4 ou 5, caractérisé en ce que comme enzyme, il contient le neuramidinase, le pronase, la papaine, le triglycéridlipase, le carboxylesterase, le cholestérinesterase, le sphingomyélinase, le phospholipase ou un mélange de ces enzymes.